# EUROPÄISCHE PATENTSCHRIFT

(11) **EP 2 872 618 B1**
(45) Veröffentlichungstag und Bekanntmachung des Hinweises auf die Patenterteilung: **19.12.2018**
(21) Anmeldenummer: 13736529.2
(22) Anmeldetag: 05.07.2013
(51) Int. Cl.: C12N 5/071

(54) **VERFAHREN ZUR KONTROLLIERTEN BEWEGUNG VON MOTILEN ZELLEN IN FLÜSSIGEN ODER GASFÖRMIGEN MEDIEN**
METHOD FOR THE CONTROLLED MOVEMENT OF MOTILE CELLS IN LIQUID OR GASEOUS MEDIA
PROCÉDÉ POUR LE DÉPLACEMENT CONTRÔLÉ DE CELLULES MOBILES DANS LES MILIEUX LIQUIDES OU GAZEUX

(30) Priorität: 16.07.2012 DE 102012212427
(43) Veröffentlichungstag der Anmeldung: 20.05.2015
(73) Patentinhaber: Leibniz-Institut für Festkörper- und Werkstoffforschung Dresden e.V., 01069 Dresden (DE)
(72) Erfinder: MAGDANZ, Veronika, 01097 Dresden (DE); SANCHEZ, Samuel, 70563 Stuttgart (DE); SCHMIDT, Oliver, G., 01219 Dresden (DE)
(74) Vertreter: Rauschenbach, Marion
(86) Internationale Anmeldenummer: PCT/EP2013/064222
(87) Internationale Veröffentlichungsnummer: WO 2014/012801

(56) Entgegenhaltungen:
- ANSLINGER K ET AL: "Application of sperm-specific antibodies for the separation of sperm from cell mixtures", FORENSIC SCIENCE INTERNATIONAL: GENETICS SUPPLEMENT SERIES, ELSEVIER IRELAND LTD, Bd. 1, Nr. 1, 1. August 2008 (2008-08-01), Seiten 394-395, XP024526846, ISSN: 1875-1768, DOI: 10.1016/J.FSIGSS.2007.10.140 [gefunden am 2008-04-25]
- ENVER KEREM DIRICAN ET AL: "Clinical outcome of magnetic activated cell sorting of non-apoptotic spermatozoa before density gradient centrifugation for assisted reproduction", JOURNAL OF ASSISTED REPRODUCTION AND GENETICS, KLUWER ACADEMIC PUBLISHERS-PLENUM PUBLISHERS, NE, Bd. 25, Nr. 8, 23. September 2008 (2008-09-23), Seiten 375-381, XP019643845, ISSN: 1573-7330, DOI: 10.1007/S10815-008-9250-1
- GALANZHA EKATERINA I ET AL: "In vivo magnetic enrichment and multiplex photoacoustic detection of circulating tumour cells", NATURE NANOTECHNOLOGY, NATURE PUBLISHING GROUP, GB, Bd. 4, Nr. 12, 1. Dezember 2009 (2009-12-01), Seiten 855-860, XP002594057, ISSN: 1748-3395, DOI: 10.1038/NNANO.2009.333 [gefunden am 2009-11-15]
- JHA R K ET AL: "Smart RISUG: A potential new contraceptive and its magnetic field-mediated sperm interaction", INTERNATIONAL JOURNAL OF NANOMEDICINE 2009 DOVE MEDICAL PRESS LTD. NZL, Bd. 4, Nr. 1, 2009, Seiten 55-64, XP002711748, ISSN: 1176-9114
- VERONIKA MAGDANZ ET AL: "Development of a Sperm-Flagella Driven Micro-Bio-Robot", ADVANCED MATERIALS, vol. 25, no. 45, 1 December 2013 (2013-12-01), pages 6581-6588, XP055172822, ISSN: 0935-9648, DOI: 10.1002/adma.201302544

## Beschreibung

Die Erfindung bezieht sich auf die Gebiete der Werkstoffwissenschaften, der Biologie und der Medizin und betrifft ein nichttherapeutisches Verfahren zur kontrollierten Bewegung von motilen Zellen in flüssigen oder gasförmigen Medien sowie magnetische Partikel zur therapeutischen Anwendung an motilen Zellen, wie sie beispielsweise bei der in-vivo- oder in-vitro-Fertilisation angewandt werden können.

Es sind verschiedene Verfahren bekannt, im Falle von geringer Mobilität von Zellen, beispielsweise Spermien, die Fertilisation sowohl im Körper als auch außerhalb, zu erreichen und/oder ihre Erfolgsrate zu verbessern.

Es ist ein Verfahren bekannt, bei dem ein Ei oder ein Embryo mit einer Schicht aus magnetischen Partikeln versehen wird und durch Anlegen eines magnetischen Feldes über einen Permanentmagneten oder einen Elektromagneten das Ei oder der Embryo in die Gebärmutter transportiert und dort stabilisiert wird (US 6,695,766 B4). Dazu werden magnetische Partikel auf ihrer Oberfläche mit reaktiven Gruppen versehen. Die magnetischen Partikel können einen Durchmesser von 0,1µm bis 200 µm aufweisen. Diese magnetischen Partikel werden mit dem Ei oder dem Embryo zusammengebracht, so dass die reaktiven Gruppen der magnetischen Partikel mit den reaktiven Gruppen auf der Oberfläche des Ei's oder des Embryo's reagieren können.

Verfahren, bei denen magnetische Partikel in der Größenordnung von Nanometern verwendet werden, haben den Nachteil, dass die magnetischen Partikel von den Zellen aufgenommen werden können und so, insbesondere bei Spermienzellen, eine Toxizität verursachen (Subiai et al., Environ.Sci.Technol. 2011, Vol.45; Rojanathathanes, et al., Fertility and Sterility, 2007, Vol.91).

Weiterhin sind Verfahren zur Herstellung von magnetischen Mikroröhrchen bekannt, die mittels Aufrollen von dünnen magnetischen Schichten hergestellt werden (Mei, Y.F., et al., Adv Mater., 2008, Vol.20). Dazu wird beispielsweise ein Glassubstrat mit einem Photolack beschichtet, dieser für 7 Sekunden mit UV-Licht bestrahlt, wobei mittels einer Schablone quadratische Strukturen erzeugt und die unerwünschten Photolackbereiche mit einem Lösungsmittel entfernt werden. Das Substrat mit den Photolackstrukturen wird mit magnetischen Materialien beschichtet und wiederum in ein Lösungsmittel gegeben, wodurch die Photolackstrukturen nun entfernt werden (Opferschicht), was zum Aufrollen der Ti/Fe-Schichten zu Mikroröhrchen führt.

Ebenfalls ist aus Anslinger K. et al: Forensic Science International: Genetics Supplement Series, Elsevier Irland Ltd. Bd. 1, Nr. 1, 1. August 2009, S. 394-395 die Abtrennung von Spermien aus einer Zellmischung bekannt. Dies wird erreicht, indem magnetische Partikel in Form von "Dynabeads", die mit einer Schicht aus spermienspezifischen Antikörpern (Pan Mouse IgG) umgeben sind, in die Zellmischung eingebracht werden. Die Spermien koppeln sich an das Beschichtungsmaterial der Dynabeads. Nachfolgend wird die Zellmischung für 3 min an einen Magneten angelegt. Dadurch werden die magnetischen Partikel vom Magneten angezogen oder abgestoßen und reichern sich in einem Bereich der Zellmischung an, so dass sie dort abgetrennt werden können. Dynabeads, wie sie in D1 eingesetzt werden, sind superparamagnetische Polystyren-Kügelchen (beads).

Weiter ist aus der Veröffentlichung Enver Kerem Dirican et al: J. of Assisted Reproduction and Genetics, Kluwer Academic Publishers-Plenum Publishers,NE, Bd. 25, Nr. 8, 23.September 2008, S. 375 - 381 ein Verfahren zur Sortierung von toten und nicht-toten Spermien bekannt, um den Prozentsatz an Spermien mit einer normalen Morphologie zu verbessern. Dabei werden die nicht-toten Spermien in einer Zellmischung abgetrennt, indem die nicht-toten Spermien an Antikörper gekoppelt werden, die sich als Schicht auf der Oberfläche von magnetischen Mikro- oder Nanopartikeln befinden. Durch das angelegte magnetische Feld werden die an die magnetischen Partikel über Antikörper gekoppelten nicht-toten Spermien von allen anderen Zellen abgetrennt.

Weiter ist ein Verfahren zur Markierung und zum Auffinden von Krebszellen bekannt (Galanzha E. et al: Nature Nanotechnology, Nature Publishing Group, GB, Bd. 4, Nr. 12, 1. Dezember 2009, S. 855-860). Die Markierung erfolgt über magnetische Partikel, die an der Oberfläche mit PEG und Human-AFT beschichtet sind. Die Kopplung der magnetischen Partikel an die Krebszellen erfolgt über das PEG und das Human-AFT und die so gekoppelten Krebszellen sind dann über photoakustische Detektion auffindbar.

Aufgabe der vorliegenden Erfindung ist es, ein nichttherapeutisches Verfahren zur kontrollierten Bewegung von motilen Zellen in flüssigen oder gasförmigen Medien und magnetische Partikel zur therapeutischen Anwendung an motilen Zellen anzugeben, mit denen die Aktivität und gesteuerte Mobilität von motilen Zellen verbessert und die Aufnahme von zellfremden Materialien möglichst verhindert wird.

Die Aufgabe wird durch die in den Ansprüchen angegebene Erfindung gelöst. Vorteilhafte Ausgestaltungen sind Gegenstand der Unteransprüche.

Bei dem erfindungsgemäßen nichttherapeutischen Verfahren zur kontrollierten Bewegung von motilen Zellen in flüssigen oder gasförmigen Medien werden eine oder mehrere motile Zellen in oder an ein oder mehrere magnetische Partikel in Form einer Röhre, eines Stabes, eines unregelmäßig geformten Hohlkörpers oder eines Netzwerkes eingebracht oder angebracht, und nachfolgend werden durch Anlegen eines externen Magnetfeldes die magnetischen Partikel mit den in oder an ihnen eingebrachten oder angebrachten motilen Zellen gerichtet bewegt.

Vorteilhafterweise wird als flüssiges Medium Wasser, biologische Flüssigkeiten und/oder Blut, und als gasförmiges Medium Luft eingesetzt.

Ebenfalls vorteilhafterweise werden als motile Zellen Spermazellen eingesetzt.

Weiterhin vorteilhafterweise werden magnetische Partikel aus ferromagnetischen oder paramagnetischen Materialien eingesetzt.

Und auch vorteilhafterweise werden magnetische Partikel aus Titan, Platin Eisen, Eisenoxid, Gold oder Glas oder aus Legierungen oder Verbindungen dieser Materialien eingesetzt, wobei nichtmagnetische Partikel mit magnetischen Materialien beschichtet eingesetzt werden.

Vorteilhaft ist es auch, wenn magnetische Partikel eingesetzt werden, die auf ihrer inneren und/oder äußeren Oberfläche funktionelle Gruppen aufweisen, wie Mannosyl/Kohlenhydrat- Gruppen oder Sperma-bindende Proteine.

Ebenfalls vorteilhaft ist es, wenn magnetische Partikel eingesetzt werden, deren Abmessungen an die Größe der motilen Zelle oder Zellen angepasst oder nur geringfügig größer oder kleiner ausgebildet ist, wobei noch vorteilhafterweise magnetische Partikel eingesetzt werden, deren Abmessungen 200 nm bis mehrere Mikrometer betragen.

Und auch vorteilhaft ist es, wenn als magnetische Partikel Mikroröhrchen oder aufgerollte Mikroröhrchen eingesetzt werden.

Von Vorteil ist es weiterhin, wenn ein externes Magnetfeld durch einen Permanentmagneten oder Elektromagneten realisiert wird.

Und auch von Vorteil ist es, wenn die motilen Zellen mit den magnetischen Partikeln in einer Flüssigkeit kontrolliert bewegt werden, die sich in vitro oder in vivo befindet.

Ebenfalls von Vorteil ist es, wenn magnetische Partikel mit motilen Zellen kontrolliert bewegt werden, bei denen die motilen Zellen chemisch oder mechanisch an die magnetischen Partikel gebunden sind.

Erfindungsgemäß erfolgt die nichttherapeutische Verwendung der mit dem erfindungsgemäßen Verfahren kontrolliert bewegten motilen Zellen in flüssigen oder gasförmigen Medien im Körper eines Säugetiers oder Menschen.

Vorteilhafterweise erfolgt die Verwendung von mit dem erfindungsgemäßen Verfahren kontrolliert bewegten Spermazellen in der Gebärmutter und/oder im Eileiter.

Die erfindungsgemäßen magnetischen Partikel in Form einer Röhre, eines Stabes, eines unregelmäßig geformten Hohlkörpers oder eines Netzwerkes dienen zur therapeutischen Anwendung an motilen Zellen für die gerichtete kontrollierte Bewegung von motilen Zellen in flüssigen oder gasförmigen Medien, wobei eine oder mehrere motile Zellen in oder an ein oder mehrere magnetische Partikel eingebracht oder angebracht werden, und nachfolgend durch Anlegen eines externen Magnetfeldes die magnetischen Partikel mit den in oder an ihnen eingebrachten oder angebrachten motilen Zellen gerichtet bewegt werden.

Vorteilhafterweise erfolgt die Anwendung der magnetischen Partikel an den motilen Zellen in Wasser, biologischen Flüssigkeiten und/oder Blut als flüssigem Medien, und die Anwendung an den motilen Zellen in Luft als gasförmigem Medium.

Weiter vorteilhafterweise erfolgt die Anwendung der magnetischen Partikel an Spermazellen als motilen Zellen.

Ebenso vorteilhafterweise werden die magnetischen Partikel als ferromagnetische oder paramagnetische Partikel angewandt.

Und auch vorteilhafterweise werden als magnetische Partikel Partikel aus Titan, Platin Eisen, Eisenoxid, Gold oder Glas oder aus Legierungen oder Verbindungen dieser Materialien angewandt, wobei auch nichtmagnetische Partikel mit einer Beschichtung aus magnetischen Materialien angewandt werden.

Vorteilhaft ist es auch, wenn als magnetische Partikel Partikel angewandt werden, die auf ihrer inneren und/oder äußeren Oberfläche funktionelle Gruppen aufweisen, wie Mannosyl/Kohlenhydrat- Gruppen oder Sperma-bindende Proteine.

Weiterhin vorteilhaft ist es, wenn als magnetische Partikel Partikel angewandt werden, deren Abmessungen an die Größe der motilen Zelle oder Zellen angepasst oder nur geringfügig größer oder kleiner ausgebildet ist, vorteilhafterweise deren Abmessungen 200 nm bis mehrere Mikrometer betragen.

Ebenso vorteilhaft ist es, wenn als magnetische Partikel Partikel in Form von Mikroröhrchen oder aufgerollten Mikroröhrchen angewandt werden.

Und auch vorteilhaft ist es, wenn die Anwendung der magnetischen Partikel in einem externen Magnetfeld erfolgt, welches durch einen Permanentmagneten oder Elektromagneten realisiert ist.

Von Vorteil ist es auch, wenn die Anwendung der magnetischen Partikel an den motilen Zellen in einer Flüssigkeit erfolgt, und diese kontrolliert bewegt werden, wobei sich die motilen Zellen in vitro oder in vivo befinden.

Ebenso von Vorteil ist es, wenn die Anwendung der magnetischen Partikel an den motilen Zellen erfolgt, die chemisch oder mechanisch an die magnetischen Partikel gebunden sind.

Weiterhin von Vorteil ist es, wenn die magnetischen Partikel an motilen Zellen verwendet werden, wobei die motilen Zellen in flüssigen oder gasförmigen Medien im Körper eines Säugetiers oder Menschen gerichtet und kontrolliert bewegt werden.

Und auch von Vorteil ist es, wenn die magnetischen Partikel verwendet werden an Spermazellen und deren gerichtete und kontrollierte Bewegung in der Gebärmutter und/oder im Eileiter erfolgt.

Mit dem erfindungsgemäßen nichttherapeutischen Verfahren zur kontrollierten Bewegung von motilen Zellen in flüssigen oder gasförmigen Medien kann erstmals die Aktivität und gesteuerte Mobilität von motilen Zellen verbessert werden, ohne dass zellfremde Materialien vorliegen, die von den Zellen aufgenommen werden könnten.

Erreicht wird dies, indem die motile Zelle oder die motilen Zellen in oder an ein magnetisches Partikel eingebracht oder angebracht werden. Die Wechselwirkung zwischen den motilen Zellen und den magnetischen Partikeln wird insbesondere durch die Form des magnetischen Partikels realisiert, welches in Form einer Röhre, eines Stabes, einer Kugel, einer Hohlkugel, eines unregelmäßig geformten Körpers, eines unregelmäßig geformten Hohlkörpers oder eines Netzwerkes eingesetzt wird. Dabei werden vorteilhafterweise magnetische Partikel mit Abmessungen eingesetzt, die an die Größe der motilen Zelle oder Zellen angepasst und nur geringfügig größer oder geringfügig kleiner ausgebildet sind, also im Wesentlichen Abmessungen im Mikrometerbereich. Noch vorteilhafterweise werden als magnetische Partikel aufgerollte Mikroröhrchen eingesetzt.

Als Materialien der magnetischen Partikel können vorteilhafterweise Eisen, Eisenoxid, Kobalt, Titan, Platin, Gold oder Glas oder Legierungen oder Verbindungen dieser Materialien eingesetzt werden, Im Falle, dass diese Materialien selbst nicht oder nicht ausreichend magnetische Eigenschaften aufweisen, sind sie mit magnetischen Materialien ganz oder teilweise umhüllt.
Es ist weiterhin vorteilhaft, wenn die magnetischen Partikel auf ihrer inneren und/oder äußeren Oberfläche funktionelle Gruppen aufweisen. Diese funktionellen Gruppen können mit funktionellen Gruppen auf der Oberfläche der motilen Zellen interagieren und/oder reagieren und so eine physikalische und/oder chemische Verbindung herstellen, die eine bessere Haftung der motilen Zellen am und im magnetischen Partikel hervorruft und damit einen sicheren Transport des magnetischen Partikels mit der oder den motilen Zellen ermöglicht.
Es ist aber ebenso möglich, dass die innere und/oder äußere Oberfläche der magnetischen Partikel so gestaltet ist, dass eine mechanische Verbindung zwischen dem magnetischen Partikel und den motilen Zellen realisiert wird, beispielsweise durch eine raue Oberfläche der magnetischen Partikel.
Weiterhin können die magnetischen Partikel auch asymmetrisch oder konisch, beispielsweise als konisch geformtes Mikroröhrchen, geformt sein.

Durch die erfindungsgemäße Lösung ist es erstmals möglich, die Bewegung von motilen Zellen zu kontrollieren, das heißt ihre Bewegung in eine gewünschte Richtung zu steuern.

Unter motilen Zellen sollen im Rahmen dieser Erfindung Zellen verstanden werden, die selbst die Fähigkeit aufweisen sich in flüssigen oder gasförmigen Medien zu bewegen. Nachdem die motile Zelle oder die motilen Zellen in oder an ein oder mehrere magnetische(s) Partikel und in das flüssige oder gasförmige Medium eingebracht worden sind, kann ein externes magnetisches Feld angelegt werden und über die Bewegung dieses Feldes die Bewegungsrichtung der magnetischen Partikel mit den motilen Zellen kontrolliert gesteuert werden. Ein derartiges externes magnetisches Feld kann durch einen Permanentmagneten oder einen Elektromagneten erzeugt werden.

Besonders vorteilhaft kann das erfindungsgemäße Verfahren zur Unterstützung der natürlichen in-vivo Fertilisation angewandt werden, wenn Spermien eine verringerte Motilität aufweisen. Dadurch wird eine erhöhte Erfolgsrate bei Befruchtungen erreicht und es wird eine alternative Reproduktionstechnik eröffnet, da eine Entnahme der Eizelle aus dem Uterus nicht notwendig ist. Die magnetischen Partikel mit Spermazellen können direkt in die Gebärmutter und die Eileiter eingebracht werden. Durch die Anwendung eines externen Magneten wird die Spermazelle damit gezielt zur Eizelle gelenkt und es kommt zur Befruchtung.

Das erfindungsgemäße Verfahren ist aber auch einsetzbar für die in-vitro Fertilisation mit dem Transport der Zellen zur Eizelle außerhalb des Körpers.

Der Vorteil der erfindungsgemäßen Lösung besteht insbesondere darin, dass die magnetischen Partikel nicht von den Zellen aufgenommen werden können und daher keine negativen Auswirkungen auf die Zellen haben.

Nachfolgend wird die Erfindung an einem Ausführungsbeispiel näher erläutert.

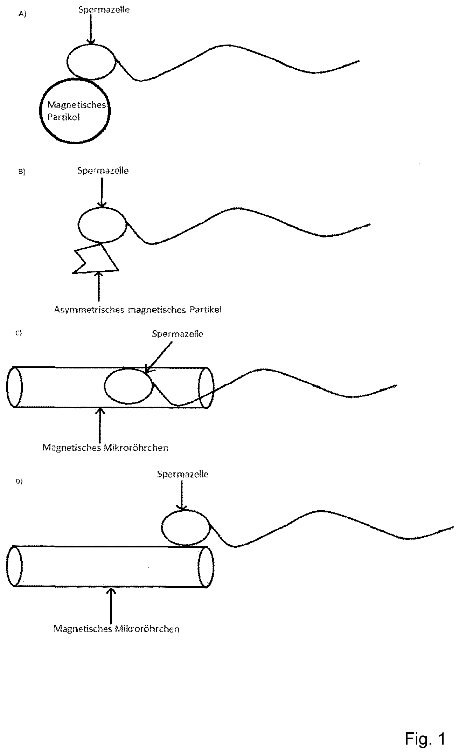

Dabei zeigt
- Fig. 1: Möglichkeiten einer Anordnung einer Spermazelle an oder in einem magnetischen Partikel in schematischer Form

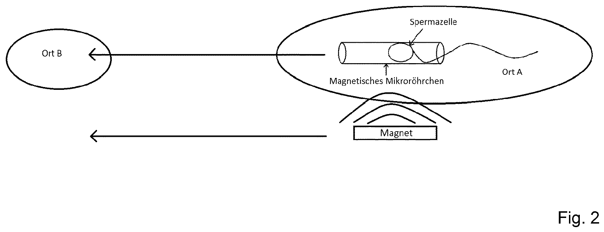

- Fig. 2: die schematische Funktionsweise der kontrollierten Bewegung einer Spermazelle in einem Mikroröhrchen

### Beispiel

10⁵ Mikroröhrchen aus einer äußeren Schicht von 5nm Titan, einer mittleren 5nm dicken Schicht aus Eisen und einer inneren 1nm dicken Platinschicht, wobei die Mikroröhrchen jeweils einem inneren Durchmesser von 5 µm und einer Länge von 50 µm aufweisen und mittels Aufrolltechnik hergestellt worden sind. Sie werden gemeinsam mit 50 µll Sperma in 3 ml Human Tubal Fluid (HTF) Medium vermischt. Dabei interagieren die Spermazellen mit den Mikroröhrchen, heften sich an deren äußere Oberfläche und/oder dringen in sie ein. Nach 5 min sind die Mikroröhrchen mit einer oder bis zu drei Spermazellen verbunden. Durch die Anwendung von Magneten können gewünschte Kombinationen aus Spermazellen und Mikroröhrchen unter dem Mikroskop selektiert werden. Etwa 100 Mikroröhrchen die an Spermazellen gebunden sind, werden durch die Bewegung des externen Magneten gezielt in eine räumlich getrennte Region gebracht. Diese Suspension wird in die Gebärmutter eingespritzt. Ein Permanentmagnet wird außerhalb des Körpers aufgelegt und über die Bewegung des Permanentmagneten wird die Bewegung der Mikroröhrchen zum Eileiter und den Eizellen gesteuert. Der erfolgreiche Transport der Spermazelle mit dem Röhrchen wird über Ultraschall verfolgt. Durch die Öffnungen der Mikroröhrchen können die Spermien mit den Eizellen funktional interagieren und die Eizellen befruchten. Nachdem die Spermazelle in die Eizelle eingedrungen ist, werden durch Bewegung des Permanentmagneten die Mikroröhrchen wieder aus dem Körper entfernt.

## Patentansprüche

1. Nichttherapeutisches Verfahren zur kontrollierten Bewegung von motilen Zellen in flüssigen oder gasförmigen Medien, bei dem eine oder mehrere motile Zellen in oder an ein oder mehrere magnetische Partikel in Form einer Röhre, eines Stabes, eines unregelmäßig geformten Hohlkörpers oder eines Netzwerkes eingebracht oder angebracht werden, und nachfolgend durch Anlegen eines externen Magnetfeldes die magnetischen Partikel mit den in oder an ihnen eingebrachten oder angebrachten motilen Zellen gerichtet bewegt werden.

2. Verfahren nach Anspruch 1, bei dem als flüssiges Medium Wasser, biologische Flüssigkeiten und/oder Blut, und als gasförmiges Medium Luft eingesetzt wird.

3. Verfahren nach Anspruch 1, bei dem als motile Zellen Spermazellen eingesetzt werden.

4. Verfahren nach Anspruch 1, bei dem magnetische Partikel aus ferromagnetischen oder paramagnetischen Materialien eingesetzt werden.

5. Verfahren nach Anspruch 1, bei dem magnetische Partikel aus Titan, Platin Eisen, Eisenoxid, Gold oder Glas oder aus Legierungen oder Verbindungen dieser Materialien eingesetzt werden, wobei nichtmagnetische Partikel mit magnetischen Materialien beschichtet eingesetzt werden.

6. Verfahren nach Anspruch 1, bei dem magnetische Partikel eingesetzt werden, die auf ihrer inneren und/oder äußeren Oberfläche funktionelle Gruppen aufweisen, wie Mannosyl/Kohlenhydrat- Gruppen oder Sperma-bindende Proteine.

7. Verfahren nach Anspruch 1, bei dem magnetische Partikel eingesetzt werden, deren Abmessungen an die Größe der motilen Zelle oder Zellen angepasst oder nur geringfügig größer oder kleiner ausgebildet ist, vorteilhafterweise deren Abmessungen 200 nm bis mehrere Mikrometer betragen.

8. Verfahren nach Anspruch 1, bei dem als magnetische Partikel Mikroröhrchen oder aufgerollte Mikroröhrchen eingesetzt werden.

9. Verfahren nach Anspruch 1, bei dem ein externes Magnetfeld durch einen Permanentmagneten oder Elektromagneten realisiert wird.

10. Verfahren nach Anspruch 1, bei dem die motilen Zellen mit den magnetischen Partikeln in einer Flüssigkeit kontrolliert bewegt werden, die sich in vitro oder in vivo befindet.

11. Verfahren nach Anspruch 1, bei dem magnetische Partikel mit motilen Zellen kontrolliert bewegt werden, bei denen die motilen Zellen chemisch oder mechanisch an die magnetischen Partikel gebunden sind.

12. Nichttherapeutische Verwendung des Verfahrens nach Anspruch 1 zur kontrollierten Bewegung von motilen Zellen in flüssigen oder gasförmigen Medien im Körper eines Säugetiers oder Menschen.

13. Verwendung nach Anspruch 12 zur kontrollierten Bewegung von Spermazellen in der Gebärmutter und/oder im Eileiter.

14. Magnetische Partikel in Form einer Röhre, eines Stabes, eines unregelmäßig geformten Hohlkörpers oder eines Netzwerkes zur therapeutischen Anwendung an motilen Zellen für die gerichtete kontrollierte Bewegung von motilen Zellen in flüssigen oder gasförmigen Medien, wobei eine oder mehrere motile Zellen in oder an ein oder mehrere magnetische Partikel eingebracht oder angebracht werden, und nachfolgend durch Anlegen eines externen Magnetfeldes die magnetischen Partikel mit den in oder an ihnen eingebrachten oder angebrachten motilen Zellen gerichtet bewegt werden.

15. Magnetische Partikel zur Anwendung nach Anspruch 14, wobei die Anwendung an den motilen Zellen in Wasser, biologischen Flüssigkeiten und/oder Blut als flüssigem Medien, und die Anwendung an den motilen Zellen in Luft als gasförmigem Medium erfolgt.

16. Magnetische Partikel zur Anwendung nach Anspruch 14, deren die Anwendung an Spermazellen als motilen Zellen erfolgt.

17. Magnetische Partikel zur Anwendung nach Anspruch 14, die als ferromagnetische oder paramagnetische Partikel angewandt werden.

18. Magnetische Partikel zur Anwendung nach Anspruch 14, die als magnetische Partikel aus Titan, Platin Eisen, Eisenoxid, Gold oder Glas oder aus Legierungen oder Verbindungen dieser Materialien angewandt werden, wobei auch nichtmagnetische Partikel mit einer Beschichtung aus magnetischen Materialien angewandt werden.

19. Magnetische Partikel zur Anwendung nach Anspruch 14, die als magnetische Partikel angewandte werden, die auf ihrer inneren und/oder äußeren Oberfläche funktionelle Gruppen aufweisen, wie Mannosyl/Kohlenhydrat- Gruppen oder Sperma-bindende Proteine.

20. Magnetische Partikel zur Anwendung nach Anspruch 14, die als magnetische Partikel angewandt werden, deren Abmessungen an die Größe der motilen Zelle oder Zellen angepasst oder nur geringfügig größer oder kleiner ausgebildet ist, vorteilhafterweise deren Abmessungen 200 nm bis mehrere Mikrometer betragen.

21. Magnetische Partikel zur Anwendung nach Anspruch 14, die als magnetische Partikel in Form von Mikroröhrchen oder aufgerollten Mikroröhrchen angewandt werden.

22. Magnetische Partikel zur Anwendung nach Anspruch 14, deren Anwendung in einem externen Magnetfeld erfolgt, welches durch einen Permanentmagneten oder Elektromagneten realisiert ist.

23. Magnetische Partikel zur Anwendung nach Anspruch 14, deren Anwendung an den motilen Zellen in einer Flüssigkeit erfolgt, und die kontrolliert bewegt werden, wobei sich die motilen Zellen in vitro oder in vivo befinden.

24. Magnetische Partikel zur Anwendung nach Anspruch 14, deren Anwendung an den motilen Zellen erfolgt, die chemisch oder mechanisch an die magnetischen Partikel gebunden sind.

25. Magnetische Partikel zur Verwendung nach Anspruch 14 an motilen Zellen, wobei die motilen Zellen in flüssigen oder gasförmigen Medien im Körper eines Säugetiers oder Menschen gerichtet und kontrolliert bewegt werden.

26. Magnetische Partikel zur Verwendung nach Anspruch 25, wobei die motilen Zellen Spermazellen sind und deren gerichtete und kontrollierte Bewegung in der Gebärmutter und/oder im Eileiter erfolgt.

## Claims

1. Non-therapeutic method for the controlled movement of motile cells in liquid or gaseous media, in which method one or more motile cells are introduced into or attached to one or more magnetic particles in the form of a tube, a rod, an irregularly shaped hollow body or a network, and the magnetic particles with the motile cells introduced therein or attached thereto are subsequently moved in a directed manner by means of application of an external magnetic field.

2. Method according to Claim 1, in which the liquid medium used is water, biological fluids and/or blood and the gaseous medium used is air.

3. Method according to Claim 1, in which the motile cells used are sperm cells.

4. Method according to Claim 1, in which magnetic particles composed of ferromagnetic or paramagnetic materials are used.

5. Method according to Claim 1, in which magnetic particles composed of titanium, platinum, iron, iron oxide, gold or glass or composed of alloys or compounds of these materials are used, with non-magnetic particles being used with a coating of magnetic materials.

6. Method according to Claim 1, in which magnetic particles comprising functional groups, such as mannosyl/carbohydrate groups or sperm-binding proteins, on their inner and/or outer surface are used.

7. Method according to Claim 1, in which magnetic particles of dimensions that are matched to the size of the motile cell or cells or are only slightly larger or smaller, advantageously of dimensions that are from 200 nm to several micrometres, are used.

8. Method according to Claim 1, in which the magnetic particles used are microtubes or rolled-up microtubes.

9. Method according to Claim 1, in which an external magnetic field is realized by a permanent magnet or electromagnet.

10. Method according to Claim 1, in which the motile cells are moved in a controlled manner with the magnetic particles in a liquid which is situated in vitro or in vivo.

11. Method according to Claim 1, in which magnetic particles containing motile cells, in which the motile cells are chemically or mechanically bound to the magnetic particles, are moved in a controlled manner.

12. Non-therapeutic use of the method according to Claim 1 for the controlled movement of motile cells in liquid or gaseous media in the body of a mammal or human.

13. Use according to Claim 12 for the controlled movement of sperm cells in the uterus and/or in the fallopian tube.

14. Magnetic particles in the form of a tube, a rod, an irregularly shaped hollow body or a network for therapeutic use on motile cells for the directed controlled movement of motile cells in liquid or gaseous media, wherein one or more motile cells are introduced into or attached to one or more magnetic particles, and the magnetic particles with the motile cells introduced therein or attached thereto are subsequently moved in a directed manner by means of application of an external magnetic field.

15. Magnetic particles for the use according to Claim 14, wherein the use is effected on the motile cells in water, biological fluids and/or blood as liquid media, and the use is effected on the motile cells in air as gaseous medium.

16. Magnetic particles for the use according to Claim 14, which are used on sperm cells as motile cells.

17. Magnetic particles for the use according to Claim 14, which are used as ferromagnetic or paramagnetic particles.

18. Magnetic particles for the use according to Claim 14, which are used as magnetic particles composed of titanium, platinum, iron, iron oxide, gold or glass or composed of alloys or compounds of these materials, with non-magnetic particles also being used with a coating composed of magnetic materials.

19. Magnetic particles for the use according to Claim 14, which are used as magnetic particles comprising functional groups, such as mannosyl/carbohydrate groups or sperm-binding proteins, on their inner and/or outer surface.

20. Magnetic particles for the use according to Claim 14, which are used as magnetic particles of dimensions that are matched to the size of the motile cell or cells or are only slightly larger or smaller, advantageously of dimensions that are from 200 nm to several micrometres.

21. Magnetic particles for the use according to Claim 14, which are used as magnetic particles in the form of microtubes or rolled-up microtubes.

22. Magnetic particles for the use according to Claim 14, which are used in an external magnetic field which is realized by a permanent magnet or electromagnet.

23. Magnetic particles for the use according to Claim 14, which are used on the motile cells in a liquid and which are moved in a controlled manner, with the motile cells being situated in vitro or in vivo.

24. Magnetic particles for the use according to Claim 14, which are used on the motile cells which are chemically or mechanically bound to the magnetic particles.

25. Magnetic particles for the use according to Claim 14 on motile cells, wherein the motile cells are moved in a directed and controlled manner in liquid or gaseous media in the body of a mammal or human.

26. Magnetic particles for the use according to Claim 25, wherein the motile cells are sperm cells and their directed and controlled movement is effected in the uterus and/or in the fallopian tube.

## Revendications

1. Procédé non thérapeutique pour le déplacement contrôlé de cellules motiles dans des milieux liquides ou gazeux, selon lequel une ou plusieurs cellules motiles sont introduites dans ou appliquées sur une ou plusieurs particules magnétiques sous la forme d'un tube, d'une barre, d'un corps creux de forme irrégulière ou d'un réseau, puis, par application d'un champ magnétique extérieur, les particules magnétiques dans lesquelles les cellules motiles ont été introduites ou sur lesquelles les cellules motiles ont été appliquées sont déplacées de manière dirigée.

2. Procédé selon la revendication 1, selon lequel de l'eau, des liquides biologiques et/ou du sang sont utilisés en tant que milieu liquide, et de l'air est utilisé en tant que milieu gazeux.

3. Procédé selon la revendication 1, selon lequel des spermatozoïdes sont utilisés en tant que cellules motiles.

4. Procédé selon la revendication 1, selon lequel des particules magnétiques en matériaux ferromagnétiques ou paramagnétiques sont utilisées.

5. Procédé selon la revendication 1, selon lequel des particules magnétiques en titane, platine, fer, oxyde de fer, or ou verre ou en alliages ou composés de ces matériaux sont utilisées, des particules non magnétiques recouvertes avec des matériaux magnétiques étant utilisées.

6. Procédé selon la revendication 1, selon lequel des particules magnétiques qui comprennent des groupes fonctionnels sur leur surface intérieure et/ou extérieure, tels que des groupes mannosyle/hydrate de carbone ou des protéines se liant au sperme, sont utilisées.

7. Procédé selon la revendication 1, selon lequel des particules magnétiques dont les dimensions sont adaptées à la taille de la ou des cellules motiles ou qui sont configurées uniquement légèrement supérieures ou inférieures, avantageusement dont les dimensions sont de 200 nm à plusieurs micromètres, sont utilisées.

8. Procédé selon la revendication 1, selon lequel des microtubes ou des microtubes enroulés sont utilisés en tant que particules magnétiques.

9. Procédé selon la revendication 1, selon lequel un champ magnétique extérieur est réalisé par un aimant permanent ou un électroaimant.

10. Procédé selon la revendication 1, selon lequel les cellules motiles sont déplacées de manière contrôlée avec les particules magnétiques dans un liquide, qui se trouve in vitro ou in vivo.

11. Procédé selon la revendication 1, selon lequel des particules magnétiques comprenant des cellules motiles sont déplacées de manière contrôlée, les cellules motiles étant reliées chimiquement ou mécaniquement aux particules magnétiques.

12. Utilisation non thérapeutique du procédé selon la revendication 1 pour le déplacement contrôlé de cellules motiles dans des milieux liquides ou gazeux dans le corps d'un mammifère ou d'un homme.

13. Utilisation selon la revendication 12 pour le déplacement contrôlé de spermatozoïdes dans l'utérus et/ou dans la trompe de Fallope.

14. Particules magnétiques sous la forme d'un tube, d'une barre, d'un corps moulé de forme irrégulière ou d'un réseau pour l'application thérapeutique à des cellules motiles pour le déplacement contrôlé dirigé de cellules motiles dans des milieux liquides ou gazeux, une ou plusieurs cellules motiles étant introduites dans ou appliquées sur une ou plusieurs particules magnétiques, puis, par application d'un champ magnétique extérieur, les particules magnétiques dans lesquelles les cellules motiles ont été introduites ou sur lesquelles les cellules motiles ont été appliquées étant déplacées de manière dirigée.

15. Particules magnétiques destinées à l'application selon la revendication 14, l'application aux cellules motiles ayant lieu dans de l'eau, des liquides biologiques et/ou du sang en tant que milieu liquide, et l'application aux cellules motiles ayant lieu dans de l'air en tant que milieu gazeux.

16. Particules magnétiques destinées à une application selon la revendication 14, dont l'application a lieu à des spermatozoïdes en tant que cellules motiles.

17. Particules magnétiques destinées à une application selon la revendication 14, qui sont utilisées en tant que particules ferromagnétiques ou paramagnétiques.

18. Particules magnétiques destinées à une application selon la revendication 14, qui sont utilisées en tant que particules magnétiques en titane, platine, fer, oxyde de fer, or ou verre ou en alliages ou composés de ces matériaux, des particules non magnétiques munies d'un revêtement en matériaux magnétiques étant également utilisées.

19. Particules magnétiques destinées à une application selon la revendication 14, qui sont utilisées en tant que particules magnétiques qui comprennent des groupes fonctionnels sur leur surface intérieure et/ou extérieure, tels que des groupes mannosyle/hydrate de carbone ou des protéines se liant au sperme.

20. Particules magnétiques destinées à une application selon la revendication 14, qui sont utilisées en tant que particules magnétiques dont les dimensions sont adaptées à la taille de la ou des cellules motiles ou qui sont configurées uniquement légèrement supérieures ou inférieures, avantageusement dont les dimensions sont de 200 nm à plusieurs micromètres.

21. Particules magnétiques destinées à une application selon la revendication 14, qui sont utilisées en tant que particules magnétiques sous la forme de microtubes ou de microtubes enroulés.

22. Particules magnétiques destinées à une application selon la revendication 14, dont l'application a lieu dans un champ magnétique extérieur qui est réalisé par un aimant permanent ou un électroaimant.

23. Particules magnétiques destinées à une application selon la revendication 14, dont l'application aux cellules motiles a lieu dans un liquide et qui sont déplacées de manière contrôlée, les cellules motiles se trouvant in vitro ou in vivo.

24. Particules magnétiques destinées à une application selon la revendication 14, dont l'application a lieu sur des cellules motiles qui sont reliées chimiquement ou mécaniquement aux particules magnétiques.

25. Particules magnétiques destinées à une application selon la revendication 14 à des cellules motiles, les cellules motiles étant déplacées de manière dirigée et contrôlée dans des milieux liquides ou gazeux dans le corps d'un mammifère ou d'un homme.

26. Particules magnétiques destinées à une utilisation selon la revendication 25, les cellules motiles étant des spermatozoïdes et leur déplacement dirigé et contrôlé ayant lieu dans l'utérus et/ou dans la trompe de Fallope.
